# EUROPEAN PATENT SPECIFICATION

(11) **EP 0 765 471 B1**
(45) Date of publication and mention of the grant of the patent: **28.05.2003**
(21) Application number: 95912531.1
(22) Date of filing: 01.03.1995
(51) Int. Cl.: G01N 21/84, G01N 33/46, G01B 11/30

(54) **ARRANGEMENT AND METHOD FOR THE DETECTION OF DEFECTS IN TIMBER**
ANORDNUNG UND VERFAHREN ZUM NACHWEIS VON FEHLERN IM HOLZ
AGENCEMENT ET PROCEDE DE DETECTION DE DEFAUTS DANS LE BOIS

(30) Priority: 08.03.1994 SE 9400849
(43) Date of publication of application: 02.04.1997
(73) Proprietor: IVP INTEGRATED VISION PRODUCTS AB, 583 35 Linköping (SE)
(72) Inventor: ASTRÖM, Anders, S-582 47 Linköping (SE); ASTRAND, Erik, S-583 31 Linköping (SE)
(74) Representative: Egeröd, Lisbeth
(86) International application number: SE9500212
(87) International publication number: WO95024636

(56) References cited:
- DE-A- 4 104 501
- US-A- 3 976 384

## Description

The present invention relates to an arrangement for the detection of defects in timber comprising at least one linear light source for illuminating a wood surface capable of moving relative to the light source and a light sensor arranged above the aforementioned wood surface.

The invention also relates to a method for the detection of defects in timber, in which a timber object is illuminated by at least one linear light source and an image is detected by a sensor arranged above the timber object.

Within the sawmill and joinery industry, for example, the identification of defective timber in an effective fashion, after sawing and also before further processing in a second stage, has long been a major problem; sorting is still a partially manual operation in many places. The criteria for what constitutes defects can vary in many cases depending on the area of application of the timber, which makes it important to be able to identify the type of defects present in a piece of timber.

Systems are certainly now available for the automatic identification of timber with certain defects, although these systems have a limited capacity to detect defects. Previously disclosed systems are able at the present time to detect darker cracks, resin pockets, larger cracks and pith edges in timber, although they are unable to detect light knots and cracks satisfactorily. The previously disclosed systems are also unable to distinguish between a resin pocket or a pith edge that is detected.

It is for instance known from US 3,976,384 to radiate light into a surface of the timber and scrutinize with light detector means the light emerging from the timber at a location spaced apart from the region of entry. Defects in the timber between the region of entry and the detection region will be indicated by the emerging light.

Known methods to measure three-dimensional shape are also described in SE 463 638 and Johannesson M. "Sheet-of-light range imaging", Licentiate thesis LIU-TEK-LIC-1993:46, Linköping University, 1993.

The object of the present invention is to make available an arrangement for the detection of defects in wood, which, by means of a combination of linear light sources and a sensor, solves the above problem, and is at least capable of detecting knots, cracks, resin pockets and pith edges. The invention is based on the so-called Tracheid effect, which means that light striking a wooden surface is scattered into the wood, and in so doing creates a light area around the point at which the incident light strikes the surface. The light is scattered differently depending on the direction of the wood fibres and depending on the presence of defects in the wood. Either by studying the intensity of the scattered light, or by comparing the scattered light with the directly reflected light, it is possible to determine whether a defect is present.

The object of the invention is met in that each light source is linear, in that the field of vision of the sensor covers an area next to a line of light illuminated by each light source, in that the sensor is two-dimensional and so arranged as to detect the presence of scattered light in the wood in that area, and in that the sensor is linked to image processing and signal processing circuits which are so arranged as to identify defects in the timber on the basis of the light scatter detected in the wood.

In order to reduce the required image processing, each light source is arranged in accordance with one particular feature of the invention essentially in the same plane as the sensor, the field of vision of which is arranged symmetrically about the illuminated line and a number of image elements are added together in a longitudinal sense on both sides of the lines.

According to a further particular feature of the invention, the sensor is also so arranged as to detect reflected light from the aforementioned lines of light, and the image processing and signal processing circuits are so arranged as to identify defects in the timber on the basis of a combination of the detected light scatter and the light reflected from the lines of light.

In accordance with one preferred embodiment of the invention, the arrangement comprises two linear light sources, which are arranged to either side of the timber in the longitudinal sense of the timber and at such a distance in relation to one another in the direction of relative movement between the wood surface and the light sources that they appear as separate lines when they are detected in the sensor, in conjunction with which the image processing and signal processing circuits are so arranged as to make comparisons between these two detected lines of light.

A second object of the invention is to make available a method for the detection of defects in timber, which permits the detection of a number of different defects. This object is achieved in that light scattered in the wood in an area around a line of light generated by each light source is detected in a sensor, and in that the detected light scatter is utilized in image processing and signal processing circuits for the purpose of identifying defects in the timber.

In accordance with one preferred characteristic of this method, the light scatter is detected in an area lying symmetrically about the illuminated line.

In accordance with one particular characteristic of this method, reflected light is detected from the aforementioned line of light in the sensor, in conjunction with which both the detected light scatter and the light reflected from the line of light are utilized in the image processing and signal processing circuits and a number of image elements are added together in a longitudinal sense on both sides of the lines for the purpose of identifying defects in the timber.

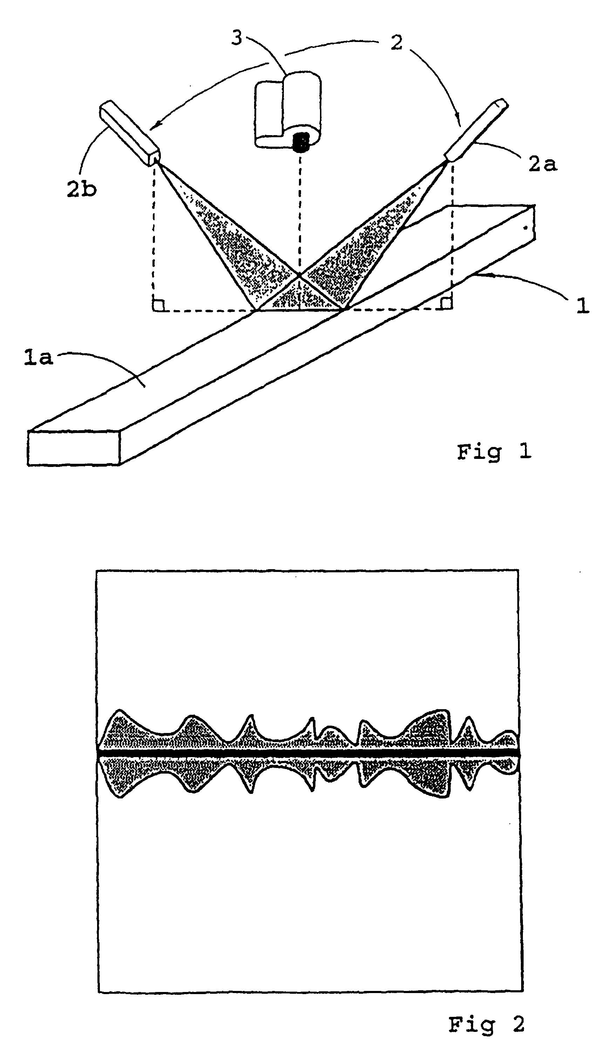

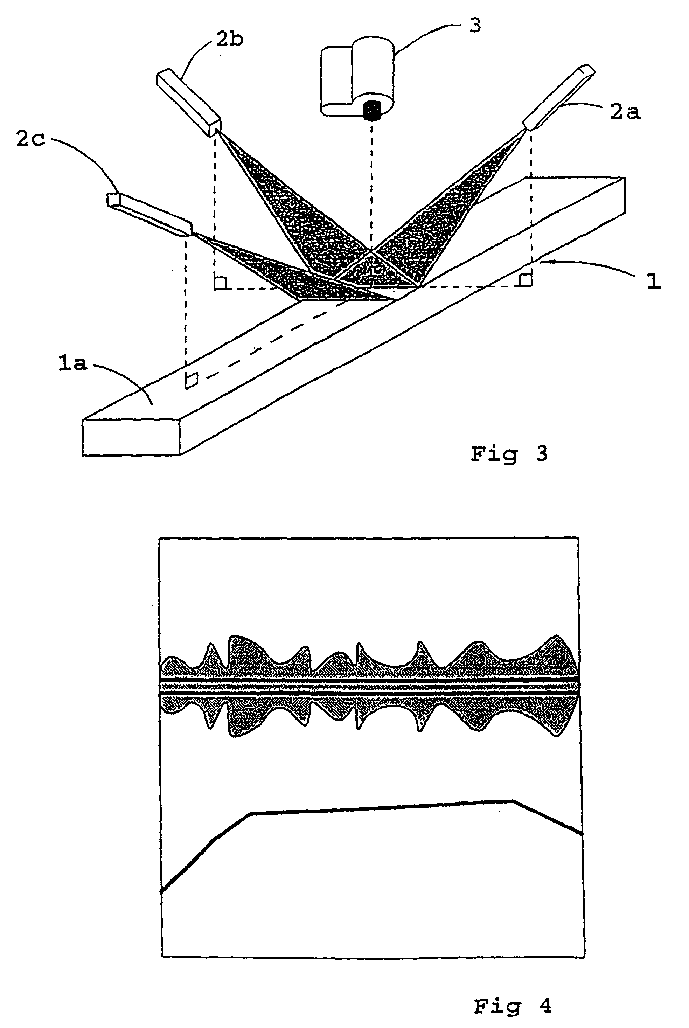

The invention is described below in greater detail with reference to the accompanying drawings, in which Figures 1 and 3 illustrate different embodiments of the invention, and Figures 2 and 4 show the image that is projected onto the sensor surface for these embodiments.

In the embodiment shown in Fig. 1, a long, sawn wooden object 1 is illuminated by two linear light sources 2a, 2b. The light sources 2a, 2b are arranged in the longitudinal sense of the object and are angled towards the wooden object in such a way as to provide uniform illumination of it. The light sources 2a, 2b are also arranged at a certain distance from one another and in the same plane as a sensor 3 arranged vertically above the wooden object 1. This sensor 3 is arranged so that it lies directly above the point at which the beams from the two light sources meet. The wooden object 1 is fed preferably in the longitudinal direction, although it is naturally also possible to feed the wooden object in the transverse direction, or to cause the light sources and the sensor to move in relation to the piece of timber.

The sensor 3 has the ability to detect both the light scattered from the light sources in the wooden object 1 and the light reflected directly on the wooden object 1. The image perceived by the sensor can be appreciated from Fig. 2. In order to compensate for the fact that the intensity of the light differs considerably between the scatter image perceived by the sensor 3 and the reflection image, the sensor must, for the purpose of detecting the scatter image, collect light from a larger area than that used for detecting the reflection image. If a sensor of identical size is used for all the image elements, this can be achieved by adding together a number of image elements in a longitudinal sense on both sides of the direct line. Another alternative way of compensating for the lower light intensity is to make the exposure time longer for the image elements struck by the scattered light, than for the image elements struck by the direct light. A third alternative is to use one sensor for detecting the light reflected directly on the surface of the wood, and a second sensor for detecting the light scattered in the wood.

In the image of the timber that can be obtained by studying the intensity of the light scattered in the timber, the so-called Tracheid effect, it is possible readily to distinguish cracks, both light and dark knots, and pith edges. This can be performed, for example, by causing the image processing and signal processing circuits to detect objects of a predetermined type in the received image. This can be performed in a simple fashion by comparing the light intensity at each point with a predetermined value. With the help of more advanced image analysis, high reliability of detection can be achieved, and the type of defect can even be classified.

In a preferred embodiment of the invention, the intensity of the directly reflected line of light can also be utilized to detect defects in the wood. In the image supplied by the sensor 3, which is obtained on the basis of the directly reflected light, it is possible to detect cracks, knots, pith edges and resin pockets using the arrangement in accordance with Fig. 1, on condition that the defects are not very light. It is nevertheless difficult to classify the various types of defect simply by using this grey-scale image as the starting point.

If, on the other hand, a combination of the image generated by the light scatter and the image generated by the direct reflection is used, it is possible by image analysis to determine the type of defects present in the timber. The image generated by the Tracheid effect essentially shows only knots and pith edges, which appear as very dark areas in the image supplied by the sensor. The light scatter associated with these types of defect differs from that in the surrounding wood, which makes it easy to identify the type of defect present in the timber. The information from this image can then be combined with the information in the ordinary image, so that it is possible with a good degree of accuracy to determine what types of defect are present in the timber.

In the arrangement illustrated in Fig. 3, the linear light sources 2a, 2b must be arranged in such a way in relation to one another that the lines of light formed lie adjacent to one another at a distance such that they appear as separate lines when they are detected in the sensor. By comparing the intensity of the light reflected on the wood surface by these light sources, it is possible to detect both the presence of wane edges on the timber and surface irregularities.

By introducing a third linear light source 2c in accordance with the arrangement shown in Fig. 3, it is possible to obtain a three-dimensional profile of the timber. By comparing the intensity of the light sources arranged on the side of the timber with the intensity of this third light source, it is possible to detect any deviations in the direction of the fibre in the timber. When a less accurate analysis of the characteristics of the timber is required, it is also possible, of course, to utilize only the image generated by the direct reflection.

In a preferred embodiment of the present invention, use is made of a two-dimensional sensor which contains circuits for parallel image processing and signal processing, for example the so-called MAPP2200 sensor. This type of sensor is preferred, as it permits the detection and processing of both the directly reflected light in a line of light and the light scattered in the timber due to the Tracheid effect. The sensor offers, amongst other things, the opportunity to combine a number of image elements on each side of the line of light, and to make comparisons between the total intensity achieved and the intensity of the line of light. Other types of sensors are entirely possible, of course, and the invention is in no way dependent on the aforementioned sensor.

## Claims

1. Arrangement for detecting defects in timber comprising at least one light source (2) for illuminating a timber surface (1 a) capable of moving relative to the light source and a light sensor (3) arranged above the timber surface (1a), **characterized in that**
each light source (2) is linear;
the sensor (3) is two-dimensional and has a field of view covering an area next to a line of light illuminated by each light source (2);
the sensor (3) is arranged to detect the presence of scattered light in the timber in said area; and
the sensor (3) is linked to image processing and signal processing circuits arranged to identify defects in the timber on the basis of the light scatter detected in the timber.

2. Arrangement in accordance with Patent Claim 1, **characterized in that** the sensor (3) is so arranged as to detect reflected light from the aforementioned lines of light, and **in that** the image processing and signal processing circuits are so arranged as to identify defects in the timber on the basis of a combination of the detected light scatter and the light reflected from the lines of light.

3. Arrangement in accordance with Patent Claim 1 or 2, **characterized in that** each light source (2) and the sensor are arranged essentially in the same plane, the field of vision of the sensor being arranged symmetrically about the line illuminated by each light source and that a number of image elements are added together in a longitudinal sense on both sides of the lines.

4. Arrangement in accordance with Patent Claim 2 or 3, **characterized in that** the exposure time is longer for image elements struck by the scattered light, than for image elements struck by the reflected light.

5. Arrangement in accordance with any of the Patent Claims 1 - 4, **characterized in that** it comprises two linear light sources (2a, 2b), which are arranged to either side of the timber in the longitudinal sense of the timber and at such a distance in relation to one another in the direction of relative movement between the wood surface and the light sources that they appear as separate lines when they are detected in the sensor (3), and **in that** the image processing and signal processing circuits are so arranged as to make comparisons between these two detected lines of light.

6. Arrangement in accordance to any of the Patent Claims 1 - 4 or 5, **characterized by** the introduction of an additional (third) linear light source (2c) at a certain, angle relative to the viewing direction of the sensor, making it possible to obtain a three-dimensional profile of the timber.

7. Method of detecting defects in timber, in which a timber object (1) is illuminated by at least one light source (2) and an image is detected by a sensor (3) arranged above the timber object (1), **characterized in that** each light source is linear, light scattered in the wood in an area around a line of light generated by each linear light source is detected by a two dimensional sensor (3), and the detected light scatter is utilized in image processing and signal processing circuits for the purpose of identifying defects in the timber.

8. Method according to claim 7, **characterized in that** light reflected from the aforementioned line of light is detected in the sensor (3), and **in that** both the detected light scatter and the light reflected from the line of light are utilized in the image processing and signal processing circuits for the purpose of identifying defects in the timber.

9. Method in accordance with claim 7 or 8, **characterized in that** the light scatter is detected in an area lying symmetrically about the line illuminated by each light source and that a number of image elements are added together in a longitudinal sense on both sides of the lines.

10. Method in accordance with claim 8 or 9, **characterized in that** the exposure time is longer for image elements struck by the scattered light, than for image elements struck by the reflected light.

## Patentansprüche

1. Vorrichtung zum Erfassen von Fehlern in Holz mit wenigstens einer Lichtquelle (2) zum Beleuchten einer Holzfläche (1a), die relativ zu der Lichtquelle beweglich ist, und einem Lichtsensor (3), der oberhalb der Holzfläche (1a) angeordnet ist, **dadurch gekennzeichnet, daß** jede Lichtquelle (2) linear ist, der Sensor (3) zweidimensional ist und ein Blickfeld besitzt, das einen Bereich neben einer Lichtlinie abdeckt, die durch jede Lichtquelle (2) beleuchtet ist, der Sensor (3) so ausgebildet ist, daß er das Auftreten von Streulicht in dem Holz in diesem Bereich erfaßt, und der Sensor (3) mit bildverarbeitenden und signalverarbeitenden Schaltungen verbunden ist, die so ausgebildet sind, daß sie Fehler in dem Holz auf der Grundlage der in dem Holz erfaßten Lichtstreuung identifizieren.

2. Vorrichtung nach Anspruch 1, **dadurch gekennzeichnet, daß** der Sensor (3) so ausgebildet ist, daß er das von den zuvor erwähnten Lichtlinien reflektierte Licht erfaßt und die bildverarbeitenden und signalverarbeitenden Schaltungen Fehler in dem Holz auf der Grundlage einer Kombination der erfaßten Lichtstreuung und des von den Lichtlinien reflektierten Lichts identifiziert.

3. Vorrichtung nach Anspruch 1 oder 2, **dadurch gekennzeichnet, daß** jede Lichtquelle (2) und der Sensor im wesentlichen in der gleichen Ebene angeordnet sind, das Blickfeld des Sensors symmetrisch um die von jeder Lichtquelle beleuchtete Linie liegt und eine Anzahl von Bildelementen im Sinne der Längsrichtung auf beiden Seiten der Linien zusammengefügt sind.

4. Vorrichtung nach Anspruch 2 oder 3, **dadurch gekennzeichnet, daß** die Einwirkzeit für die von dem gestreuten Licht getroffenen Bildelemente länger als für die von dem reflektierten Licht getroffenen Bildelemente ist.

5. Vorrichtung nach einem der Ansprüche 1 bis 4, **dadurch gekennzeichnet, daß** sie zwei lineare Lichtquellen (2a, 2b) aufweist, die auf jeweils einer Seite des Holzes im Sinne der Längsrichtung des Holzes und in einem solchen Abstand in Bezug zueinander in der Richtung der relativen Bewegung zwischen der Holzfläche und den Lichtquellen angeordnet sind, daß sie als separate Linien erscheinen, wenn sie in dem Sensor (3) erfaßt werden, und die bildverarbeitenden und signalverarbeitenden Schaltungen so ausgebildet sind, daß sie Vergleiche zwischen diesen beiden erfaßten Lichtlinien anstellen.

6. Vorrichtung nach einem der Ansprüche 1 bis 4 oder 5, **dadurch gekennzeichnet, daß** durch das Hinzufügen einer zusätzlichen (dritten) linearen Lichtquelle (2c) in einem bestimmten Winkel relativ zu der Blickrichtung des Sensors es ermöglicht ist, ein dreidimensionales Profil des Holzes zu erhalten.

7. Verfahren zur Erfassung von Fehlern in Holz, bei welchem ein Holzgegenstand (1) mit wenigstens einer Lichtquelle (2) beleuchtet und ein Bild von einem Sensor (3) erfaßt wird, der oberhalb des Holzgegenstandes (1) angeordnet ist, **dadurch gekennzeichnet, daß** jeder Lichtsensor linear ist, daß in dem Holz in einem Bereich um eine Lichtlinie, die durch jeweils eine lineare Lichtquelle erzeugt wird, gestreutes Licht durch einen zweidimensionalen Sensor (3) erfaßt wird und die erfaßte Lichtstreuung in bildverarbeitenden und signalverarbeitenden Schaltungen zum Zwecke des Identifizierens von Fehlern in dem Holz verwendet wird.

8. Verfahren nach Anspruch 7, **dadurch gekennzeichnet, daß** das von der zuvor erwähnten Lichtlinie reflektierte Licht in dem Sensor (3) erfaßt wird und sowohl die erfaßte Lichtstreuung als auch das von der Lichtlinie reflektierte Licht in den bildverarbeitenden und signalverarbeitenden Schaltungen zum Zwecke des Identifizierens von Fehlern in dem Holz verwendet werden.

9. Verfahren nach Anspruch 7 oder 8, **dadurch gekennzeichnet, daß** die Lichtstreuung in einem Bereich erfaßt wird, der symmetrisch um die durch jede Lichtquelle beleuchtete Linie liegt und eine Anzahl von Bildelementen auf beiden Seiten der Linien im Sinne einer Längsrichtung zusammengefügt werden.

10. Verfahren nach Anspruch 8 oder 9, **dadurch gekennzeichnet, daß** die Einwirkzeit für die von dem gestreuten Licht getroffenen Bildelemente größer als für die von dem reflektierten Licht getroffenen Bildelemente ist.

## Revendications

1. Agencement pour détecter des défauts dans un bois d'oeuvre comportant au moins une source lumineuse (2) pour illuminer une surface de bois d'oeuvre (la) capable de se déplacer par rapport à la source lumineuse, et un capteur de lumière (3) agencé au-dessus de la surface de bois d'oeuvre (la), **caractérisé en ce que**
chaque source lumineuse (2) est linéaire,
le capteur (3) est bidimensionnel et a un champ de vue couvrant une zone proche d'une ligne de lumière illuminée par chaque source lumineuse (2),
le capteur (3) est conçu pour détecter la présence d'une lumière diffractée dans le bois d'oeuvre dans ladite zone, et
le capteur (3) est relié à de circuits de traitement d'image et de traitement de signal conçus pour identifier des défauts dans le bois d'oeuvre sur la base de la diffraction de lumière détectée dans le bois d'oeuvre.

2. Agencement selon la revendication 1, **caractérisé en ce que** le capteur (3) est conçu de manière à détecter une lumière réfléchie par les lignes de lumière mentionnées ci-dessus, et **en ce que** les circuits de traitement d'image et de traitement de signal sont conçus de manière à identifier des défauts dans le bois d'oeuvre sur la base d'une combinaison de la diffraction de lumière détectée et de la lumière réfléchie par les lignes de lumière.

3. Agencement selon la revendication 1 ou 2, **caractérisé en ce que** chaque source lumineuse (2) et le capteur sont agencés essentiellement dans le même plan, le champ de vision du capteur étant agencé de manière symétrique autour de la ligne illuminée par chaque source lumineuse et **en ce que** de nombreux éléments d'image sont ajoutés ensemble dans un sens longitudinal sur les deux côtés des lignes.

4. Agencement selon la revendication 2 ou 3, **caractérisé en ce que** le temps d'exposition pour des éléments d'image frappés par la lumière diffractée est plus long que pour des éléments d'image frappés par la lumière réfléchie.

5. Agencement selon l'une quelconque des revendications 1 à 4, **caractérisé en ce qu'**il comporte deux sources lumineuses linéaires (2a, 2b), qui sont agencées sur chaque côté du bois d'oeuvre dans le sens longitudinal du bois d'oeuvre et à une distance telle l'une par rapport à l'autre dans la direction du mouvement relatif entre la surface de bois et les sources lumineuses, qu'elles apparaissent sous forme de lignes séparées lorsqu'elles sont détectées dans le capteur (3), et **en ce que** les circuits de traitement d'image et de traitement de signal sont conçus de manière à effectuer des comparaisons entre ces deux lignes de lumière détectées.

6. Agencement selon l'une quelconque des revendications 1 à 4 ou 5, **caractérisé par** l'introduction d'une (troisième) source lumineuse linéaire supplémentaire (2c) à un certain angle par rapport à la direction d'observation du capteur, rendant possible d'obtenir un profil tridimensionnel du bois d'oeuvre.

7. Procédé de détection de défauts dans un bois d'oeuvre, dans lequel un objet de bois d'oeuvre (1) est illuminé par au moins une source lumineuse (2) et une image est détectée par un capteur (3) agencé au-dessus de l'objet de bois d'oeuvre (1), **caractérisé en ce que** chaque capteur de lumière est linéaire, la lumière diffractée dans le bois dans une zone située autour d'une ligne de lumière générée par chaque source lumineuse linéaire est détectée par un capteur bidimensionnel (3), et la diffraction de lumière détectée est utilisée dans des circuits de traitement d'image et de traitement de signal dans le but d'identifier des défauts dans le bois d'oeuvre.

8. Procédé selon la revendication 7, **caractérisé en ce qu'**une lumière réfléchie par la ligne de lumière mentionnée ci-dessus est détectée dans le capteur (3), et **en ce que** la diffraction de lumière détectée et la lumière réfléchie par la ligne de lumière sont toutes deux utilisées dans les circuits de traitement d'image et de traitement de signal dans le but d'identifier des défauts dans le bois d'oeuvre.

9. Procédé selon la revendication 7 ou 8, **caractérisé en ce que** la diffraction de lumière est détectée dans une zone se trouvant de manière symétrique autour de la ligne illuminée par chaque source lumineuse et **en ce que** de nombreux éléments d'image sont ajoutés ensemble dans un sens longitudinal sur les deux côtés des lignes.

10. Procédé selon la revendication 8 ou 9, **caractérisé en ce que** le temps d'exposition pour des éléments d'image frappés par la lumière diffractée est plus long que pour des éléments d'image frappés par la lumière réfléchie.
